# EUROPÄISCHE PATENTSCHRIFT

(11) **EP 2 465 477 B1**
(45) Veröffentlichungstag und Bekanntmachung des Hinweises auf die Patenterteilung: **06.05.2015**
(21) Anmeldenummer: 11190253.2
(22) Anmeldetag: 23.11.2011
(51) Int. Cl.: A61F 5/453

(54) **Vorrichtung zur Ableitung von unkontrolliertem Harnabgang**
Device for diverting uncontrolled urine discharge
Dispositif destiné à la déviation de l'écoulement d'urine non contrôlé

(30) Priorität: 14.12.2010 DE 202010016574 U
(43) Veröffentlichungstag der Anmeldung: 20.06.2012
(73) Patentinhaber: Jaeniche, Wilhelm, 77694 Kehl-Leutesheim (DE)
(72) Erfinder: Grundke, Reinhold, 84547 Emmerting (DE)
(74) Vertreter: Hofstetter, Schurack & Partner

(56) Entgegenhaltungen:
- DE-A1- 2 721 330
- DE-A1-102008 020 570
- US-A- 4 872 464
- US-A- 5 618 277

## Beschreibung

Die vorliegende Erfindung betrifft eine Vorrichtung zur Ableitung von unkontrolliertem Harnabgang bei männlichen Personen mit einem einteiligen Grundkörper aus Silikon, Silikonkautschuk oder einem Kunststoff, wobei der Grundkörper einen zylinderförmigen ersten Kammerabschnitt, dessen Innendurchmesser etwas geringer ist oder gleich einem Außendurchmesser des Penis des Benutzers ist, einen sich an den ersten Kammerabschnitt anschließenden zweiten Kammerabschnitt, dessen maximaler Innendurchmesser größer ist als der Innendurchmesser des ersten Kammerabschnitts sowie einen sich an den zweiten Kammerabschnitt anschließenden dritten Kammerabschnitt mit einem Schlauchanschluss aufweist, wobei der erste Kammerabschnitt an seinem dem zweiten Kammerabschnitt gegenüberliegenden Ende einen sich nach außen erweiternden, trichterförmigen und umlaufenden Kragen aufweist.

Eine derartige Vorrichtung ist aus der US-A-5 168 277 bekannt. Eine weitere, vergleichbare Vorrichtung ist aus der DE 201 13 445 U1 bekannt. Im Vergleich zu anderen bekannten Harnableitungsvorrichtungen ist die in der DE 201 13 445 U1 offenbarte Vorrichtung leicht anzubringen und gewährleistet zudem eine kontrollierte Ableitung von Harn. Allerdings hat sich in der Praxis herausgestellt, dass bei unsachgemäßer Anwendung oder bei einem übermäßigen Krafteinsatz durch den Benutzer der Vorrichtung die Dehnlaschen, welche zum Aufweiten der Vorrichtung am oberen Ende des ersten Kammerabschnitts angebracht sind, abreißen oder zumindest beschädigt werden. Ohne die Dehnlaschen ist die Vorrichtung nur schwer anbringbar. Aus der US-A-5 713 880 ist ebenfalls eine gattungsgemäße Vorrichtung zur Ableitung von unkontrolliertem Harnabgang beschrieben. Bei einigen gezeigten Ausführungsbeispielen dieser bekannten Vorrichtung ist an deren oberem Ende ein umstülparer zylinder- beziehungsweise röhrenförmiger Kammerabschnitt ausgebildet. Am oberen Ende dieses zylinder- beziehungsweise röhrenförmigen Abschnitts ist zudem ein umlaufender Verstärkungsring ausgebildet. Nachteilig an dieser bekannten Vorrichtung ist jedoch, dass es mittels des röhrenförmigen Kammerabschnitts nicht möglich ist, den darunter liegenden ersten Kammerabschnitt, der wiederum einen geringeren Innendurchmesser aufweist, genügend zu dehnen, um das Einführen des Penis in die Vorrichtung zu erleichtern. Der röhrenförmige Abschnitt der bekannten Vorrichtung wird daher in bevorzugter Weise in aufgerolltem Zustand bereitgestellt. Eine Dehnung des ersten Abschnitts ist aber auch dadurch nicht möglich, da durch das Aufrollen der röhrenförmige Kammerabschnitt relativ starr wird.

Es ist Aufgabe der vorliegenden Erfindung, eine Vorrichtung zur Ableitung von unkontrolliertem Harnabgang bei männlichen Personen der eingangs genannten Art bereitzustellen, die eine sichere und einfache Anbringung der Vorrichtung an dem Penis des Benutzers, eine damit verbundene bessere Handhabung und einen besseren Tragekomfort sowie eine verbesserte Stabilität der Vorrichtung gewährleistet.

Zur Lösung dieser Aufgabe dient eine gattungsgemäße Vorrichtung gemäß den Merkmalen des Anspruchs 1.

Vorteilhafte Ausgestaltungen der Erfindung sind in den Unteransprüchen beschrieben.

Eine erfindungsgemäße Vorrichtung zur Ableitung von unkontrolliertem Harnabgang bei männlichen Personen weist einen einteiligen Grundkörper aus Silicon, Siliconkautschuk oder einem Kunststoff auf, wobei der Grundkörper einen zylinderförmigen ersten Kammerabschnitt, dessen Innendurchmesser etwas geringer oder gleich einem Außendurchmesser des Penis des Benutzers ist, einen sich an den ersten Kammerabschnitt anschließenden zweiten Kammerabschnitt, dessen maximaler Innendurchmesser größer ist als der Innendurchmesser des ersten Kammerabschnitts sowie einen sich an den zweiten Kammerabschnitt anschließenden dritten Kammerabschnitt mit einem Schlauchanschluss aufweist. Der erste Kammerabschnitt weist an seinem dem zweiten Kammerabschnitt gegenüberliegenden Ende einen sich nach außen erweiternden, trichterförmigen und umlaufenden Kragen auf. Durch die Ausgestaltung eines sich nach außen erweiternden und trichterförmigen umlaufenden Kragens ist es dem Benutzer möglich, durch Anfassen des Kragens und ein entsprechendes Ziehen an dem Kragen nach außen, d.h. weg von der Vorrichtung, den ersten Kammerabschnitt derart zu dehnen, dass ein einfaches und sicheres Einführen des Penis in die Vorrichtung gewährleistet ist. Zudem ist durch die umlaufende Ausgestaltung des Kragens eine Beschädigung dieses Abschnitts der erfindungsgemäßen Vorrichtung durch eine unsachgemäße Anwendung oder einen entsprechenden übermäßigen Kraftaufwand nicht möglich. Erfindungsgemäß ist die Wandstärke des zweiten Kammerabschnitts größer als die Wandstärke des ersten Kammerabschnitts. Dadurch wird zum einen die Wandstärke des ersten Kammerabschnitts auf eine für den Benutzer hinsichtlich des Tragekomforts reduzierte Wandstärke beschränkt, andererseits weist der zweite Kammerabschnitt durch die größere Wandstärke eine genügend große Stabilität auf.

In weiteren vorteilhaften Ausgestaltungen der erfindungsgemäßen Vorrichtung ist die Wandstärke des Kragens zumindest in einem Übergangsbereich zwischen dem ersten Kammerabschnitt und dem Kragen größer als die Wandstärke des ersten Kammerabschnitts. Insbesondere weist der gesamte Kragen eine größere Wandstärke auf als der angrenzende erste Kammerabschnitt. Dadurch ist gewährleistet, dass eine entsprechende Kraft auf das obere Ende des ersten Kammerabschnitts angewendet werden kann, um diesen genügend zu dehnen. Zudem ist eine Beschädigung des Kragens wie auch dem Übergangsbereich zwischen dem Kragen und der ersten Kammerabschnitt nahezu ausgeschlossen. Es ist aber auch möglich, dass in dem Übergangsbereich zwischen dem ersten Kammerabschnitt und dem Kragen ein innen und/oder außen umlaufender Verstärkungsring ausgebildet ist. Des Weiteren kann am Kragen mindestens ein Verstärkungssteg ausgebildet sein, wobei sich der Verstärkungssteg bis in den Bereich des ersten Kammerabschnitts erstreckt.

In einer weiteren vorteilhaften Ausgestaltung der erfindungsgemäßen Vorrichtung kann in einem Übergangsbereich zwischen dem ersten und dem zweiten Kammerabschnitt eine zumindest teilweise innen und/oder außen umlaufende Wandverstärkung ausgebildet sein. Auch diese Wandverstärkung dient zur Erhöhung der Stabilität der Vorrichtung insgesamt.

In weiteren vorteilhaften Ausgestaltungen der erfindungsgemäßen Vorrichtung weist der zweite Kammerabschnitt eine zylindrische, sich in Richtung des dritten Kammerabschnitts verjüngende Form auf. Es ist aber auch möglich, dass der Innendurchmesser des zweiten Kammerabschnitts größer ist als die Höhe des zweiten Kammerabschnitts entlang einer Längsachse der Vorrichtung. Bei der letztgenannten Ausführungsform ergibt sich eine etwas gedrungenere Ausbildung der Vorrichtung 10 als bei der erstgenannten Ausführungsform. Verschiedene Anwendungsbereiche verlangen jedoch eine etwas kürze Ausgestaltung der Vorrichtung in ihrer Längserstreckung.

Bei Ausführungsformen der erfindungsgemäßen Vorrichtung mit mindestens einer an dem Kragen befestigten Befestigungslasche kann die Vorrichtung an einer Unterhose oder an einem Gürtel befestigt werden. Die Befestigungslasche kann dabei einstückig mit dem Kragen verbunden sein.

In einer weiteren vorteilhaften Ausgestaltung der erfindungsgemäßen Vorrichtung ist in einem Übergangsbereich zwischen dem ersten Kammerabschnitt und dem zweiten Kammerabschnitt mindestens ein Spül- und Belüftungsanschluss ausgebildet. Dadurch ist es möglich, dass der Grundkörper der erfindungsgemäßen Vorrichtung jederzeit und einfach mittels einer Spülflüssigkeit gereinigt werden kann. Die ebenfalls ausgebildete Belüftungsfunktion erleichtert den Harnabfluss aus der Vorrichtung. In weiteren vorteilhaften Ausgestaltungen der erfindungsgemäßen Vorrichtung ist zwischen dem zweiten und dem dritten Kammerabschnitt am Außenumfang der Vorrichtung mindestens ein Verstärkungssteg ausgebildet. Der Verstärkungssteg kann dabei einstückig mit dem Grundkörper ausgebildet sein. Es ist aber auch möglich, dass der Verstärkungssteg mit dem Grundkörper verklebt oder verschweißt ist. Ein in diesem Bereich ausgebildeter Verstärkungssteg verhindert zuverlässig ein Abknicken des Schlauchanschlusses der Vorrichtung und sichert somit den kontinuierlichen Harnabfluss aus der Vorrichtung.

Weitere Einzelheiten, Merkmale und Vorteile der Erfindung ergeben sich aus den in den folgenden Zeichnungen dargestellten Ausführungsbeispielen. Es zeigen:
- Figur 1: eine schematische Darstellung einer erfindungsgemäßen Vorrichtung zur Ableitung von unkontrolliertem Harnabgang bei männlichen Personen gemäß einer ersten Ausführungsform;
- Figur 2: eine schematisch dargestellte Ansicht von oben auf die erfindungsgemäßen Vorrichtung gemäß Figur 1;
- Figur 3: eine schematische Darstellung einer erfindungsgemäßen Vorrichtung zur Ableitung von unkontrolliertem Harnabgang bei männlichen Personen gemäß einer zweiten Ausführungsform;
- Figur 4: eine schematische Schnittdarstellung der erfindungsgemäßen Vorrichtung gemäß Figur 3; und
- Figur 5: eine Detaildarstellung eines Bereichs der erfindungsgemäßen Vorrichtung gemäß den Figuren 3 und 4.

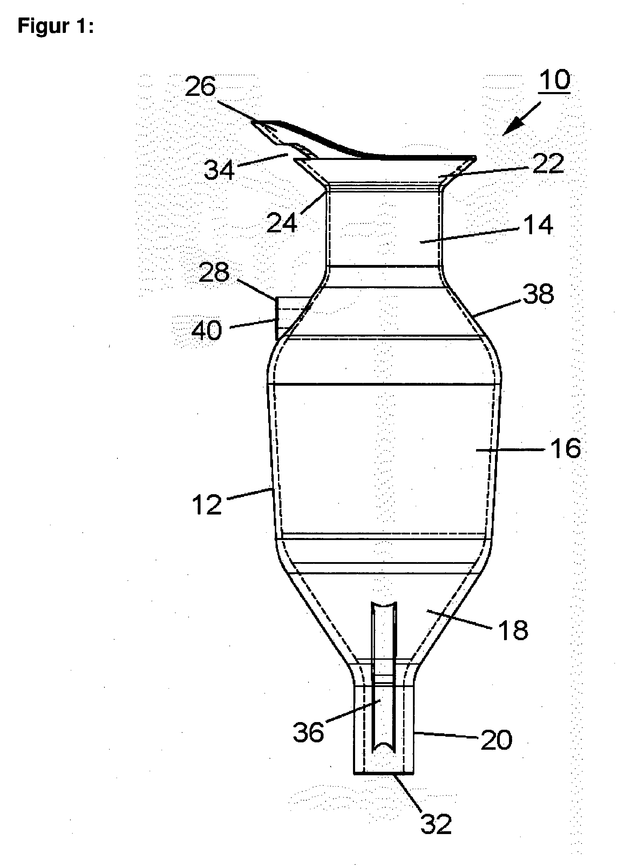

Figur 1 zeigt eine schematische Darstellung einer Vorrichtung 10 zur Ableitung von unkontrolliertem Harnabgang bei männlichen Personen. Die Vorrichtung 10 besteht dabei aus einem einteiligen Grundkörper 12 aus Silicon, Siliconkautschuk oder einem physiologisch verträglichen Kunststoff. Durch die Verwendung dieser Materialien ist einerseits eine gute Verträglichkeit, insbesondere Hautverträglichkeit gewährleistet. Zudem sind diese Materialien leicht reinigbar. Der Grundkörper 12 umfasst einen zylinderförmigen ersten Kammerabschnitt 14, dessen Innendurchmesser etwas geringer oder gleich einem Außendurchmesser des Penis des Benutzers ist, einen sich an den ersten Kammerabschnitt 14 anschließenden zweiten Kammerabschnitt 16, dessen maximaler Innendurchmesser größer ist als der Innendurchmesser des ersten Kammerabschnitts 14 sowie einen sich an den zweiten Kammerabschnitt 16 anschließenden Kammerabschnitt 18 mit einem Schlauchanschluss 20. Der Ausdruck "etwas geringer" ist so auszulegen, dass der Innendurchmesser des ersten Kammerabschnitts 14 zum Beispiel bei einem Penisdurchmesser von 22 bis 40 mm ungefähr 20 mm beträgt.

Man erkennt, dass sich der zweite Kammerabschnitt 16 in Richtung des dritten Kammerabschnitts 18 verjüngt. Der maximale Außendurchmesser des zweiten Kammerabschnitts 16 beträgt ca. 45 bis 50 mm, in dem dargestellten Ausführungsbeispiel beträgt er ca. 47 mm. Des Weiteren ist in Figur 1 schematisch angedeutet, dass sich die Wandstärken des ersten, zweiten und dritten Kammerabschnitts 14, 16, 18 voneinander unterscheiden. So ist die Wandstärke des zweiten Kammerabschnitts 16 größer als die Wandstärke des ersten Kammerabschnitts 14 ausgebildet. In dem dargestellten Ausführungsbeispiel beträgt die Wandstärke des ersten Kammerabschnitts 14 ca. 0,7 mm, die Wandstärke des zweiten Kammerabschnitts 16 beträgt ca. 1,3 mm. Die Wandstärke des dritten Kammerabschnitts 18 ist wiederum größer als die des zweiten Kammerabschnitts 16, sie beträgt in dem dargestellten Ausführungsbeispiel ca. 2 mm.

Aus der Figur 1 wird zudem deutlich, dass der erste Kammerabschnitt 14 an seinem dem zweiten Kammerabschnitt 16 gegenüberliegenden Ende einen sich nach außen erweiternden, trichterförmigen und umlaufenden Kragen 22 aufweist. Die maximale Öffnungsweite des Kragens 22 kann dabei in nicht-gedehntem Zustand zwischen 30 und 50 mm betragen. In dem dargestellten Ausführungsbeispiel beträgt die maximale Öffnungsweite in nicht-gedehntem Zustand ca. 37 mm. Zudem ist angedeutet, dass die Wandstärke des Kragens 22 größer ist als die Wandstärke des ersten Kammerabschnitts 14. In dem dargestellten Ausführungsbeispiel beträgt die Wandstärke des Kragens 22 ca. 1,0 mm. Aber auch andere Wandstärken sind denkbar. Des Weiteren kann in einem Übergangsbereich 24 zwischen dem ersten Kammerabschnitt 14 und dem Kragen 22 ein innen und/oder außen umlaufender Verstärkungsring ausgebildet sein. In Figur 1 ist eine innen umlaufende Wandverstärkung im Übergangsbereich 24 angedeutet.

Des Weiteren erkannt man, dass der Kragen 22 an seinem dem ersten Kammerabschnitt gegenüberliegenden Ende mindestens eine Befestigungslasche 26 aufweist. Die Befestigungslasche 26 ist dabei einstückig mit dem Kragen 22 ausgebildet und weist eine Öffnung beziehungsweise Öse 38 auf. Mit der Befestigungslasche 34 kann die Vorrichtung 10 an einem Gürtel oder dergleichen befestigt werden. Anstelle der Öse 34 kann natürlich auch eine andere Befestigungsvorrichtung, wie zum Beispiel ein Klettband oder Druckknöpfe an der Befestigungslasche 26 angebracht werden.

In dem dargestellten ersten Ausführungsbeispiel der Vorrichtung 10 sind zwischen dem zweiten und dem dritten Kammerabschnitt 16, 18 am Außenumfang der Vorrichtung 10 zwei Verstärkungsstege 36 ausgebildet. Diese sind einstückig mit dem Grundkörper 12 hergestellt und verhindern ein Abknicken des Schlauchanschlusses 20. In dem dargestellten Ausführungsbeispiel beträgt die Wandstärke des Anschlusses 20 ca. 4 mm. Auch dies trägt zu einer hervorragenden Stabilität des Schlauchanschlusses 20 gegenüber einem unbeabsichtigten Abknicken durch den Benutzer bei. Eine Ausführöffnung 32 des Schlauchanschlusses 20 weist dabei einen Durchmesser von ca. 8 mm gegenüber einem Außendurchmesser des Schlauchanschlusses 20 von ca. 12 mm auf.

Des Weiteren erkennt man, dass in einem Übergangsbereich 38 zwischen dem ersten Kammerabschnitt 14 und dem zweiten Kammerabschnitt 16 ein Spül- und Belüftungsanschluss 28 ausgebildet ist. Eine entsprechende Spül- und Entlüftungsöffnung 40 ist angedeutet.

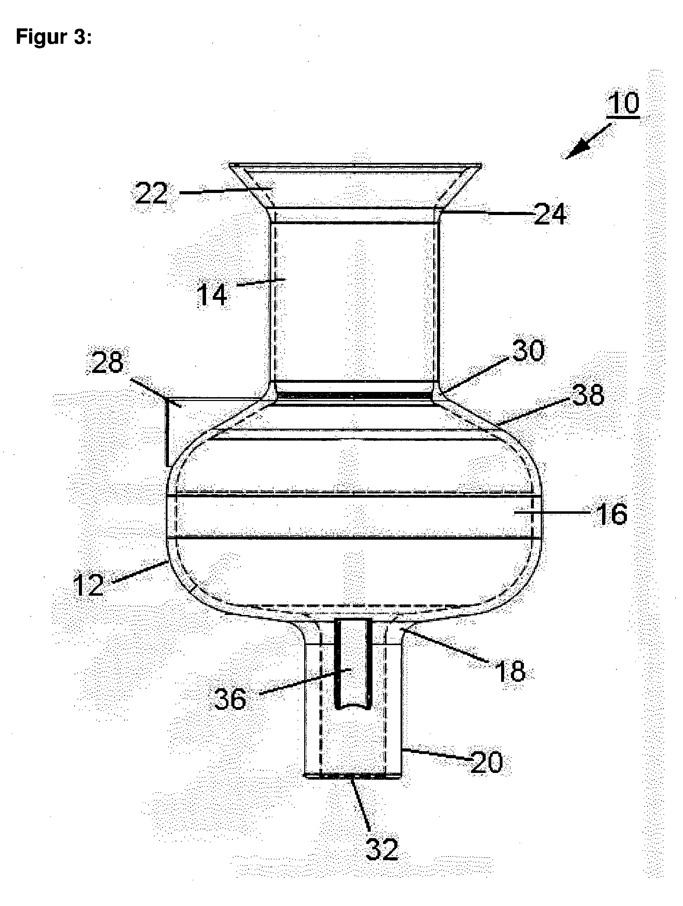

Die Dimensionen der Vorrichtung 10 können abhängig von der jeweiligen Größe des Penis eines Benutzers ausgelegt werden. Bei einer für den durchschnittlichen Benutzer ausgelegten Vorrichtung 10 beträgt der Innendurchmesser des zweiten Kammerabschnitts 16 ungefähr 35 bis 50 mm. Der erste Kammerabschnitt 14 hat gemäß dieser Ausführungsform einen Innendurchmesser von ungefähr 22 mm und einen Außendurchmesser von ungefähr 23 mm. Entsprechendes gilt auch für die in den Figuren 3 bis 5 dargestellte zweite Ausführungsform der Vorrichtung 10.

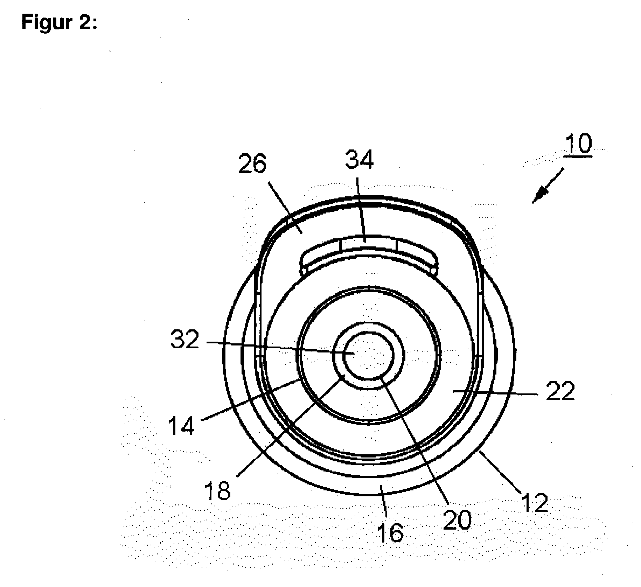

Figur 2 zeigt eine schematisch dargestellte Ansicht von oben auf die Vorrichtung 10 gemäß Figur 1. Man erkennt die umlaufende trichterförmige Ausgestaltung des Kragens 22 sowie die Anordnung der Befestigungslasche 26 mit der Öse 34 an dem Kragen 22. Des Weiteren wird deutlich, dass der Außendurchmesser des zweiten Kammerabschnitts 16 größer ist als der Außendurchmesser des ersten Kammerabschnitts 14. Die entsprechende Ausgestaltung des Schlauchabschnitts 20 mit der Ausflussöffnung 32 ist ebenfalls erkennbar.

Figur 3 zeigt eine schematische Darstellung einer Vorrichtung 10 zur Ableitung von unkontrolliertem Harnabgang bei männlichen Personen gemäß einer zweiten Ausführungsform. Im Unterschied zu der in den Figuren 1 und 2 dargestellten ersten Ausführungsform weist diese Ausführungsform der Vorrichtung 10 keine an dem Kragen 22 angeordnete Befestigungslasche 26 auf. Zudem ist die Form des zweiten Kammerabschnitts 16 anders gestaltet. Man erkennt, dass bei dieser Ausführungsform der Innendurchmesser des zweiten Kammerabschnitts 16 größer ist als die Höhe des zweiten Kammerabschnitts 16 entlang einer Längsachse der Vorrichtung 10. Dadurch ergibt sich im Vergleich zu der ersten Ausführungsform eine kürzere und gedrungenere Ausgestaltung der Vorrichtung 10 insgesamt. Bezüglich der weiteren Merkmale der in der Figur 3 dargestellten Ausführungsform der Vorrichtung 10 wird auf die Ausführungen zur ersten Ausführungsform der Vorrichtung 10 verwiesen.

Auch bei der zweiten Ausführungsform der Vorrichtung 10 weisen die unterschiedlichen Elemente der Vorrichtung 10 verschiedene Wandstärken auf. So wird wiederum deutlich, dass die Wandstärke des ersten Kammerabschnitts 14 geringer ist als die Wandstärke des zweiten Kammerabschnitts 16. Der dritte Kammerabschnitt 18 und der Schlauchanschluss 20 weisen wiederum eine größere Wandstärke auf als der zweite Kammerabschnitt 16. Auch die Wandstärke des Kragens 22 ist größer als die Wandstärke des ersten Kammerabschnitts 14. Wiederum ist in einem Übergangsbereich 24 zwischen dem ersten Kammerabschnitt 14 und dem Kragen 22 eine Wandverstärkung ausgebildet. Die Wandstärke des Kragens 22 kann dabei zwischen 0,8 und 2,0 mm, insbesondere 1,2 mm, betragen. Die Wandstärke des ersten Kammerabschnitts 14 kann zwischen 0,4 und 1,0 mm, insbesondere 0,6 mm, betragen. Der zweite Kammerabschnitt 16 weist üblicherweise eine Wandstärke zwischen 1,1 und 1,5 mm, insbesondere 1,2 mm, auf. In dem dargestellten Ausführungsbeispiel beträgt die Wandstärke des dritten Kammerabschnitts ca. 4,0 mm, gleiches gilt für den Schlauchabschnitt 20. Aber auch andere geeignete Dimensionierungen sind möglich.

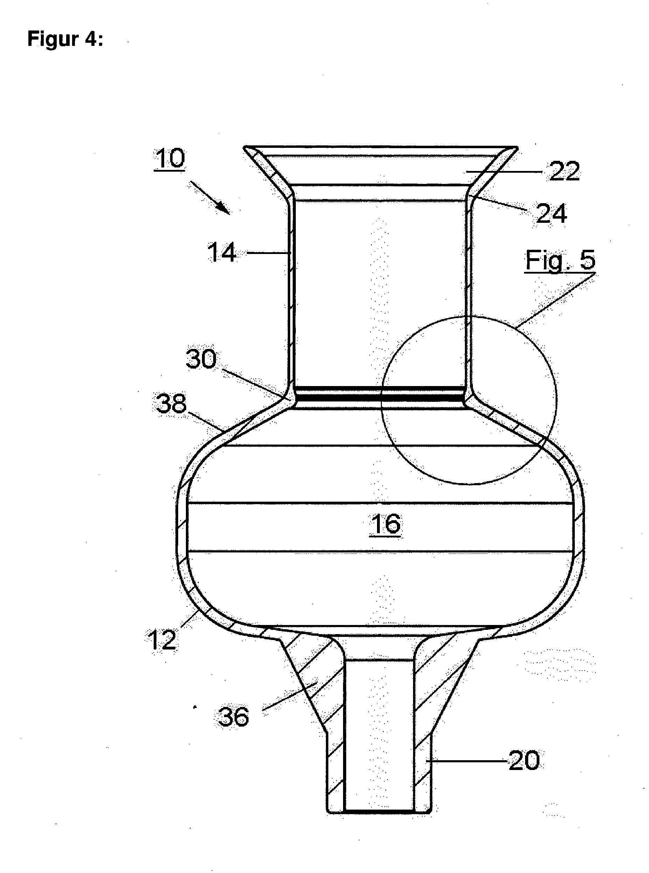

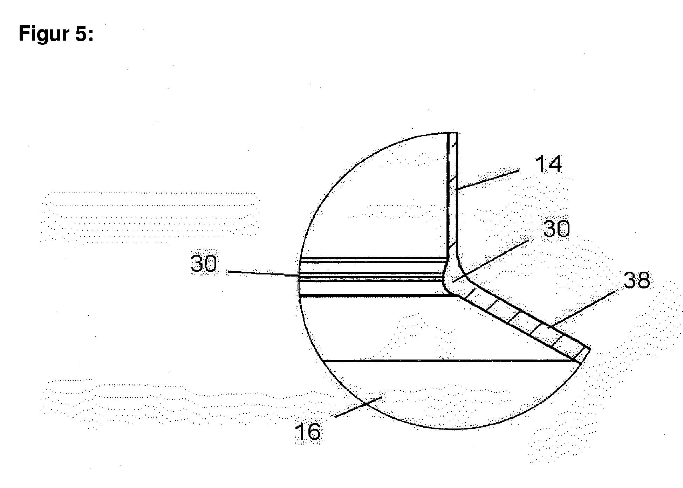

Wie auch bei dem in den Figuren 1 und 2 beschriebenen Ausführungsbeispiel der Vorrichtung 10 ist zwischen dem zweiten Kammerabschnitt 16 und dem ersten Kammerabschnitt 14 ein trichterförmiger Übergangsbereich 38 ausgebildet. Im Gegensatz zu dem ersten Ausführungsbeispiel ist bei dem zweiten Ausführungsbeispiel der Vorrichtung 10 im weiteren Übergangsbereich zwischen dem Abschnitt 38 und dem ersten Kammerabschnitt 14 eine Wandverstärkung 30 ausgebildet. Die Wandverstärkung 30 wird auch durch die in Figur 4 dargestellte Schnittdarstellung der Vorrichtung 10 sowie durch die Detaildarstellung dieses Bereichs der Vorrichtung 10 gemäß Figur 5 deutlich. Man erkennt, dass eine in diesem Bereich nach innen ragende Materialverdickung beziehungsweise Wandverstärkung 30 ausgebildet ist. Die Wandverstärkung 30 ist dabei innen in der Vorrichtung 10 umlaufend ausgebildet. Die Wandverstärkung 30 ist dabei um ca. ein Drittel größer ausgebildet als die Wandstärke des ersten Kammerabschnitts 14.

## Patentansprüche

1. Vorrichtung zur Ableitung von unkontrolliertem Harnabgang bei männlichen Personen, mit einem einteiligen Grundkörper (12) aus Silikon, Silikonkautschuk oder einem Kunststoff, wobei der Grundkörper (12) einen zylinderförmigen ersten Kammerabschnitt (14), dessen Innendurchmesser etwas geringer oder gleich einem Außendurchmesser des Penis des Benutzers ist, einen sich an den ersten Kammerabschnitt (14) anschließenden zweiten Kammerabschnitt (16), dessen maximaler Innendurchmesser größer ist als der Innendurchmesser des ersten Kammerabschnitts (14) sowie einen sich an den zweiten Kammerabschnitt (16) anschließenden dritten Kammerabschnitt (18) mit einem Schlauchanschluss (20) aufweist, wobei der erste Kammerabschnitt (14) an seinem dem zweiten Kammerabschnitt (16) abgewandten Ende einen sich nach außen erweiternden, trichterförmigen und umlaufenden Kragen (22) aufweist,
**dadurch gekennzeichnet,**
**dass** die Wandstärke des zweiten Kammerabschnitts (16) größer ist als die Wandstärke des ersten Kammerabschnittes (14).

2. Vorrichtung nach Anspruch 1,
**dadurch gekennzeichnet,**
**dass** die Wandstärke des Kragens (22) zumindest in einem Übergangsbereich (24) zwischen dem erste Kammerabschnitt (14) und dem Kragen (22) größer ist als die Wandstärke des ersten Kammerabschnittes (14).

3. Vorrichtung nach Anspruch 1 oder 2,
**dadurch gekennzeichnet,**
**dass** in einem Übergangsbereich (24) zwischen dem ersten Kammerabschnitt (14) und dem Kragen (22) ein innen und/oder außen umlaufender Verstärkungsring ausgebildet ist.

4. Vorrichtung nach einem der vorhergehenden Ansprüche,
**dadurch gekennzeichnet,**
**dass** am Kragen (22) mindestens ein Verstärkungssteg ausgebildet ist, wobei sich der Verstärkungssteg bis in den Bereich des ersten Kammerabschnitts (14) erstreckt.

5. Vorrichtung nach einem der vorhergehenden Ansprüche,
**dadurch gekennzeichnet,**
**dass** der Kragen (22) an seinem dem ersten Kammerabschnitt (14) gegenüberliegenden Ende mindestens eine Befestigungslasche (26) aufweist.

6. Vorrichtung nach Anspruch 5,
**dadurch gekennzeichnet,**
**dass** die Befestigungslasche (26) einstückig mit dem Kragen (22) ausgebildet ist.

7. Vorrichtung nach einem der vorhergehenden Ansprüche;
**dadurch gekennzeichnet,**
**dass** in einem Übergangsbereich (28) zwischen dem ersten und dem zweiten Kammerabschnitt (14, 16) eine zumindest teilweise innen und/oder außen umlaufende Wandverstärkung (30) ausgebildet ist.

8. Vorrichtung nach einem der vorhergehenden Ansprüche,
**dadurch gekennzeichnet,**
**dass** der zweite Kammerabschnitt (16) eine zylindrische, sich in Richtung des dritten Kammerabschnitts (28) verjüngende Form aufweist.

9. Vorrichtung nach einem der Ansprüche 1 bis 7,
**dadurch gekennzeichnet,**
**dass** der Innendurchmesser des zweiten Kammerabschnitts (16) größer ist als die Höhe des zweiten Kammerabschnitts (16) entlang einer Längsachse der Vorrichtung (10).

10. Vorrichtung nach einem der vorhergehenden Ansprüche,
**dadurch gekennzeichnet,**
**dass** in einem Übergangsbereich (38) zwischen dem ersten Kammerabschnitt (14) und dem zweiten Kammerabschnitt (16) mindestens ein Spül- und Belüftungsanschluss (28) ausgebildet ist.

11. Vorrichtung nach einem der vorhergehenden Ansprüche,
**dadurch gekennzeichnet,**
**dass** zwischen dem zweiten und dem dritten Kammerabschnitt (16, 18) am Außenumfang der Vorrichtung (10) mindestens ein Verstärkungssteg (36) ausgebildet ist.

12. Vorrichtung nach Anspruch 11,
**dadurch gekennzeichnet,**
**dass** der Verstärkungssteg (36) einstückig mit dem Grundkörper (12) ausgebildet ist.

13. Vorrichtung nach Anspruch 11,
**dadurch gekennzeichnet,**
**dass** der Verstärkungssteg (36) mit dem Grundkörper (12) verklebt oder verschweißt ist.

## Claims

1. Device for draining uncontrolled urination in male persons, including an integral base body (12) of silicone, silicone rubber or a plastic, wherein the base body (12) has a cylindrical first chamber section (14), the internal diameter of which is slightly less than or equal to an external diameter of the penis of the user, a second chamber section (16) adjoining the first chamber section (14), the maximum internal diameter of which is larger than the internal diameter of the first chamber section (14), as well as a third chamber section (18) adjoining the second chamber section (16) with a hose connector (20), wherein the first chamber section (14) has an outwardly expanding, funnel-shaped and circumferential collar (22) on its end facing away from the second chamber section (16),
**characterized in that**
the wall thickness of the second chamber section (16) is larger than the wall thickness of the first chamber section (14).

2. Device according to claim 1,
**characterized in that**
the wall thickness of the collar (22) is larger than the wall thickness of the first chamber section (14) at least in a transitional area (24) between the first chamber section (14) and the collar (22).

3. Device according to claim 1 or 2,
**characterized in that**
an internally and/or externally circumferential reinforcing ring is formed in a transitional area (24) between the first chamber section (14) and the collar (22).

4. Device according to any one of the preceding claims,
**characterized in that**
at least one reinforcing web is formed on the collar (22), wherein the reinforcing web extends up to the area of the first chamber section (14).

5. Device according to any one of the preceding claims,
**characterized in that**
the collar (22) has at least one fixing flap (26) on its end opposing the first chamber section (14).

6. Device according to claim 5,
**characterized in that**
the fixing flap (26) is formed integrally with the collar (22).

7. Device according to any one of the preceding claims,
**characterized in that**
an at least partially internally and/or externally circumferential wall reinforcement (30) is formed in a transitional area (28) between the first and the second chamber section (14, 16).

8. Device according to any one of the preceding claims,
**characterized in that**
the second chamber section (16) has a cylindrical shape tapering towards the third chamber section (28).

9. Device according to any one of claims 1 to 7,
**characterized in that**
the internal diameter of the second chamber section (16) is larger than the height of the second chamber section (16) along a longitudinal axis of the device (10).

10. Device according to any one of the preceding claims,
**characterized in that**
at least one flushing and venting connector (28) is formed in a transitional area (38) between the first chamber section (14) and the second chamber section (16).

11. Device according to any one of the preceding claims,
**characterized in that**
at least one reinforcing web (36) is formed on the outer circumference of the device (10) between the second and the third chamber section (16, 18).

12. Device according to claim 11,
**characterized in that**
the reinforcing web (36) is formed integrally with the base body (12).

13. Device according to claim 11,
**characterized in that**
the reinforcing web (36) is adhered or welded to the base body (12).

## Revendications

1. Appareil destiné à l'évacuation d'une émission incontrôlée d'urine chez des personnes de sexe masculin, avec un corps de base d'une seule pièce (12) en silicone, caoutchouc de silicone ou une matière synthétique, moyennant quoi le corps de base (12) présente une première section de chambre (14) de forme cylindrique, dont le diamètre intérieur est quelque peu inférieur ou égal à un diamètre extérieur du pénis de l'utilisateur, une deuxième section de chambre (16) à la suite de la première section de chambre (14), dont le diamètre intérieur maximum est supérieur au diamètre intérieur de la première section de chambre (14), ainsi qu'une troisième section de chambre (18), à la suite de la deuxième section de chambre (16) avec un raccord de tuyau (20), moyennant quoi la première section de chambre (14) présente, à son extrémité tournée vers la deuxième section de chambre (16), un collet (22) s'évasant vers l'extérieur, en entonnoir et périphérique,
**caractérisé en ce**
**que** l'épaisseur de la paroi de la deuxième section de chambre (16) est supérieure à l'épaisseur de la paroi de la première section de chambre (14).

2. Appareil selon la revendication 1,
**caractérisé en ce**
**que** l'épaisseur de la paroi du collet (22) est supérieure, au moins dans une zone de transition (24) entre la première section de chambre (14) et le collet (22), à l'épaisseur de la paroi de la première section de chambre (14).

3. Appareil selon la revendication 1 ou 2,
**caractérisé en ce**
**qu'**une bague de renforcement circonférentielle à l'intérieur et / ou à l'extérieur est constituée dans une zone de transition (24) entre la première section de chambre (14) et le collet (22).

4. Appareil selon l'une des revendications précédentes,
**caractérisé en ce**
**qu'**au moins une nervure de renforcement est constituée sur le collet (22), moyennant quoi la nervure de renforcement s'étend jusque dans la zone de la première section de chambre (14).

5. Appareil selon l'une des revendications précédentes,
**caractérisé en ce**
**que** le collet (22) présente, à son extrémité opposée à la première section de chambre (14), au moins une patte de fixation (26).

6. Appareil selon la revendication 5,
**caractérisé en ce**
**que** la patte de fixation (26) est constituée d'une seule pièce avec le collet (22).

7. Appareil selon l'une des revendications précédentes,
**caractérisé en ce**
**qu'**un renfort de paroi (30), au moins partiellement circonférentiel à l'intérieur et / ou à l'extérieur, est constitué dans une zone de transition (28) entre la première et la deuxième section de chambre (14, 16).

8. Appareil selon l'une des revendications précédentes,
**caractérisé en ce**
**que** la deuxième section de chambre (16) présente une forme cylindrique qui s'amincit dans la direction de la troisième section de chambre (28).

9. Appareil selon l'une des revendications 1 à 7,
**caractérisé en ce**
**que** le diamètre intérieur de la deuxième section de chambre (16) est supérieur à la hauteur de la deuxième section de chambre (16) le long d'un axe longitudinal de l'appareil (10).

10. Appareil selon l'une des revendications précédentes,
**caractérisé en ce**
**qu'**au moins un raccord de rinçage et d'aération (28) est constitué dans une zone de transition (38) entre la première section de chambre (14) et la deuxième section de chambre (16).

11. Appareil selon l'une des revendications précédentes,
**caractérisé en ce**
**qu'**au moins une nervure de renforcement (36) est constituée entre la deuxième et la troisième section de chambre (16, 18) à la périphérie extérieure de l'appareil (10).

12. Appareil selon la revendication 11,
**caractérisé en ce**
**que** la nervure de renforcement (36) est constituée d'une seule pièce avec le corps de base (12).

13. Appareil selon la revendication 11,
**caractérisé en ce**
**que** la nervure de renforcement (36) est collée ou soudée avec le corps de base (12).
